Europäisches Patentamt

European Patent Office

Office européen des brevets

(19)

(11) Veröffentlichungsnummer : **0 097 917**
**B1**

(12) **EUROPÄISCHE PATENTSCHRIFT**

(45) Veröffentlichungstag der Patentschrift :
05.03.86

(21) Anmeldenummer : 83106117.1

(22) Anmeldetag : 22.06.83

(51) Int. Cl.⁴ : **A 61 B  8/14**

(54) **Ultraschall-Tomographiegerät.**

(30) Priorität : 30.06.82 DE 3224464

(43) Veröffentlichungstag der Anmeldung :
11.01.84 Patentblatt 84/02

(45) Bekanntmachung des Hinweises auf die Patenterteilung : 05.03.86 Patentblatt 86/10

(84) Benannte Vertragsstaaten :
DE FR GB NL

(56) Entgegenhaltungen :
WO-A-80 /001 93
WO-A-83 /000 09
US-A- 4 105 018
US-A- 4 130 112
US-A- 4 137 777
US-A- 4 317 369

(73) Patentinhaber : **Siemens Aktiengesellschaft**
**Berlin und München Wittelsbacherplatz 2**
**D-8000 München 2 (DE)**

(72) Erfinder : **Hassler, Dieter**
**Flurweg 3**
**D-8521 Uttenreuth (DE)**
Erfinder : **Trautenberg, Elmar, Dr.**
**Steinacher Strasse 32a**
**D-8510 Fürth-Stadeln (DE)**

Jouve, 18, rue St-Denis, 75001 Paris, France

## Beschreibung

Die Erfindung bezieht sich auf ein Ultraschall-Tomographiegerät gemäß den Merkmalen des Oberbegriffs des Patentanspruchs 1.

Ein Ultraschall-Tomographiegerät für Transmissions-Tomographie (UCTT) ist z. B. aus der US-PS-41 05 018 vorbekannt. Hier erzeugt ein einzelner Ultraschallsender, der an einer konkav gekrümmten Abstrahlfläche mehrere schmale Sendewandlerelemente umfaßt, ein sich fächerförmig erweiterndes Feld von Ultraschallstrahlen. Als Ultraschall-Empfänger-Array mit einer Vielzahl von einzelnen Empfangswandlerelementen, das dem Ultraschall-Sender bezüglich des zu untersuchenden Objektes (z. B. weibliche Brust) diametral gegenüberliegend angeordnet ist. Die Anordnung aus Ultraschall-Sender- und Ultraschall-Empfänger-Array läßt sich um das zu untersuchende Objekt drehen. Man erhält so tomographische Schnittbilder aus einer Vielzahl von Blickwinkeln. Wie bei der Röntgen-Computer-Tomographie lassen sich die Intensitäten an den Kreuzpunkten der Ultraschallstrahlen in den verschiedenen Blickrichtungen und daraus dann ein Tomographie-Schnittbild rechnerisch ermitteln. Weitere Ultraschall-Tomographiegeräte sind für die Reflexions-Tomographie (UCTR) bekannt, z. B. durch den Aufsatz « Resolution And Image Quality By Ultrasonic Echo Tomography : Experimental Approach » von E. Hundt, G. Maderlechner, E. Kronmüller und E. Trautenberg aus « Fifth International Symposium on Ultrasonic Imaging and Tissue Characterization And Second International Symposium on Ultrasonic Materials Characterization », June 1-6, 1980, Seite 7, oder auch durch den Aufsatz « Ultrasonic Reflectivity Tomography : Reconstruction With Circular Transducer Arrays » von Stephen J. Norton und Melvin Linzer aus « Ultrasonic Imaging 1 », 1979, Seiten 154 bis 184.

Die Ultraschall-Transmissions-Tomographie (UCTT) und die Ultraschall-Reflexions-Tomographie (UCTR) haben jede für sich Vor- und Nachteile. Im Gegensatz zur Reflexions-Tomographie erlaubt die Transmissions-Tomographie beispielsweise die Gewinnung von Zusatzinformationen, wie z. B. örtliche Schallgeschwindigkeitsverteilung und Dämpfungsverteilung. Was die Ortsauflösung betrifft, so ist jedoch die Transmissions-Tomographie in ihrer diagnostischen Aussagekraft begrenzt. Die Reflexions-Tomographie liefert eine bessere Ortsauflösung als die Transmissions-Tomographie, wenn der technische Aufwand vertretbar sein soll.

Aufgabe vorliegender Erfindung ist es, ein Ultraschall-Reflexions-Tomographiegerät anzugeben, das bei einem einfachen technischen Aufwand eine optimale Ortsauflösung erlaubt. Das Reflexions-Tomographiegerät soll bei Bedarf ohne allzu großen technischen Mehraufwand auch für Transmissions-Tomographie einsetzbar sein.

Die Aufgabe wird erfindungsgemäß mit den kennzeichnenden Merkmalen des Patentanspruchs 1 gelöst.

Die Erfindung vereinigt in hervorragender Weise die hervorragenden Auflösungseigenschaften eines auf unterschiedliche Objekttiefen fokussierbaren Sektorabtasters mit der Möglichkeit des Einsatzes bei der Ultraschall-Reflexions-Tomographie. Damit ist mit geringem technischen Aufwand ein ausgezeichnet auflösendes Ultraschall-Tomographiegerät für Reflexions-Tomographie geschaffen. Die Konzeption dieses Reflexions-Tomographiegerätes erlaubt ohne allzu großen technischen Mehraufwand den Einsatz für Transmissions-Tomographie. Hierzu braucht neben den nach dem Sende-/Echoverfahren arbeitenden Sektorabtastern lediglich noch ein Ultraschall-Transmissionssender vorhanden zu sein, dem zum Transmissions-Tomographie-Betrieb ein Empfänger diametral gegenüberliegend zugeordnet ist. Der Transmissionssender sollte vorzugsweise ebenfalls ein Sektorabtaster sein, der jedoch jetzt linienfokussiert sein sollte. Hierdurch ergibt sich eine besonders feine Abtastung im Transmissionsbetrieb. Der Ultraschall-Empfänger für Transmissionsbetrieb ist vorzugsweise ein Ultraschall-Empfangs-Array, das zusammen mit dem Transmissionssender in ähnlicher Weise um das Untersuchungsobjekt rotierbar angeordnet ist, wie dies beim Ultraschall-Transmissions-Tomographiegerät der zuvor abgehandelten US-PS 41 05 018 der Fall ist.

Die nach dem Sende-/Echoverfahren arbeitenden und unterschiedlich fokussierten Sektorabtaster sowie gegebenenfalls ein Ultraschall-Transmissionssender können am Umfang eines Rotationselementes gegeneinander versetzt angeordnet sein. Ebensogut können auch einige oder auch alle nach dem Sende-/Echoverfahren arbeitenden und unterschiedlich fokussierten Sektorabtaster gegebenenfalls zusammen mit einem Ultraschall-Transmissionssender dicht benachbart in einem gemeinsamen Gehäuse, das auf einem Rotationskörper drehbar montiert ist, angeordnet sein.

Ein Ultraschall-Abtastgerät, das eine Mehrzahl von nach dem Sende-/Echoverfahren arbeitenden Ultraschall-Sendern/Empfängern umfaßt, die drehbar angeordnet sind, ist beispielsweise aus der US-PS 42 45 511 bekannt. Bei diesem Gerät rotieren aber die Sender/Empfänger einerseits nicht um das Untersuchungsobjekt. Es handelt sich also nicht um ein Ultraschall-Tomographiegerät wie im vorliegenden Sinne. Andererseits sind die rotierenden Ultraschall-Sender/Empfänger selbst auch keine Sektorabtaster. Alle Ultraschall-Sender/Empfänger erzeugen vielmehr gemeinsam in Verbindung mit einem im Strahlengang angeordneten ebenen Reflektor ein einzelnes Ultraschall-Sektorfeld, dessen Sektorspitze außerhalb der Ebene der auf den Reflektor geworfenen Ultraschallstrahlen liegt. Nicht die einzelnen Sender/Empfänger sind also Sektorabtaster ; vielmehr ist das Gesamtgerät selbst der Sektorabtaster, der lediglich ein einziges Sektorabtastfeld erzeugt.

Ein Ultraschall-Abtastgerät, das bereits mit einer Mehrzahl von einzelnen Sektorabtastern arbeitet, ist allerdings aus der US-PS 39 90 300 vorbekannt. Bei diesem Gerät sind aber die einzelnen Sektorabtaster auf einem Bogensegment angeordnet, das mit Hilfe eines Schlittens in horizontaler und vertikaler Richtung relativ zum Untersuchungsobjekt bewegbar ist. Eine Rotation um das Untersuchungsobjekt, d. h. eine Ultraschall-Tomographie im vorliegenden erfindungsgemäßen Sinne, ist mit diesem Gerät also nicht möglich.

Schließlich ist aus der GB-PS-13 16 549 noch ein Ultraschall-Abtastgerät bekannt, bei dem mehrere Ultraschallwandler, die unterschiedlich gestufte Betriebsfrequenzen haben, auf einem gemeinsamen Träger rotieren. Dieser Träger befindet sich mit seiner Drehachse in der Brennlinie eines zylindrischen Parabolreflektors. Die von den Ultraschallwandlern auf den Parabolreflektor abgestrahlten Ultraschallwellen werden demnach an diesem reflektiert, so daß sich ein reflektierter Ultraschallstrahl ergibt, der parallel zu sich selbst in einer Ebene verschoben wird. Das Ultraschall-Abtastgerät der GB-PS arbeitet also nicht als Sektorabtaster, sondern vielmehr als Gerät zur Erzeugung eines Linear-Scans, wobei die einzelnen Ultraschallwandler allerdings wegen der unterschiedlichen Betriebsfrequenzen auf unterschiedliche Objekttiefen fokussierbar sind.

Weitere Vorteile und Einzelheiten der Erfindung ergeben sich aus der nachfolgenden Beschreibung verschiedener Ausführungsbeispiele anhand der Zeichnung und in Verbindung mit den Unteransprüchen. Es zeigen :

Figur 1 den Prinzipaufbau der Erfindung in Draufsicht,

Figur 2 das Aufbauschema der Erfindung in perspektivischer Ansicht,

Figur 3 eine Modifikation der Ausführungsbeispiele der Figuren 1 und 2.

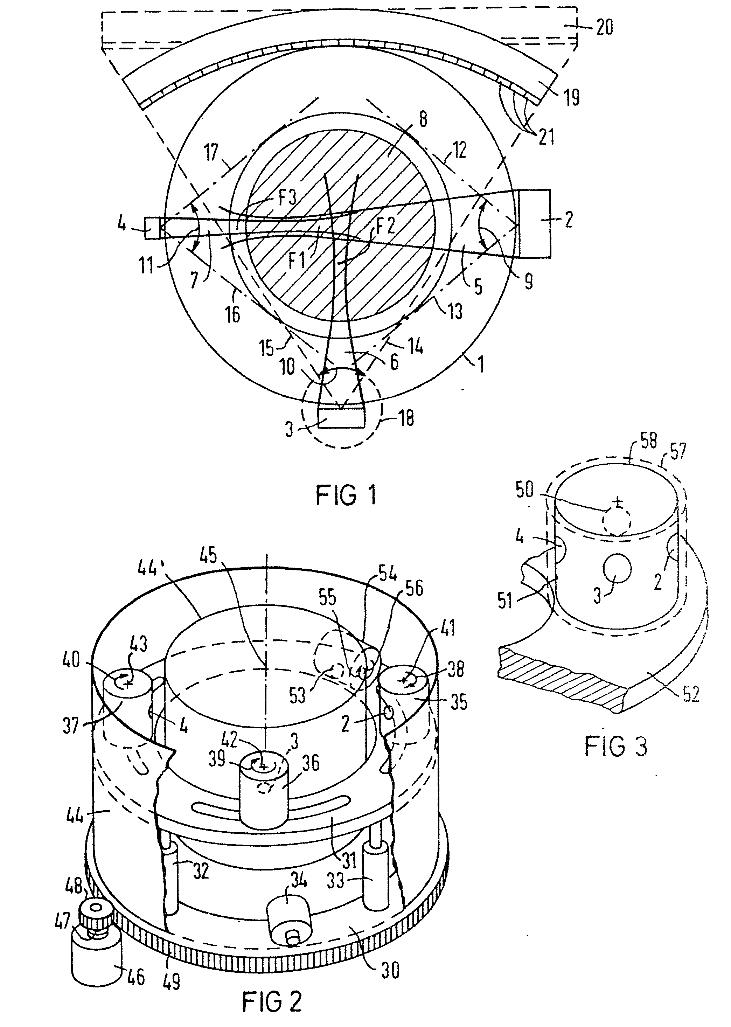

In der Figur 1 sind am Rand einer rotierbaren Ringscheibe 1 insgesamt drei mechanische Sektorabtaster 2, 3 und 4, die auf unterschiedliche Objekttiefen fokussier sind, angeordnet. Die Fokustiefen der Schallkeulen 5, 6 und 7 der einzelnen Sektorabtaster sind in der Figur 1 mit F1, F2 und F3 angedeutet. Entsprechend der Lage dieser Fokusse tastet also der Sektorabtaster 2 das Untersuchungsobjekt 8 (im vorliegenden Fall z. B. die weibliche Brust) im tiefsten Schichtgebiet ab. Entsprechend tastet der Sektorabtaster 3 das Untersuchungsobjekt in einem mittleren Tiefengebiet und der Sektorabtaster 4 das Untersuchungsobjekt im Randgebiet ab. Die unterschiedliche Fokussierung F1, F2, F3 kann in üblicher Weise wahlweise durch rein mechanische Krümmung der Sektorabtaster, durch Vorschaltung von Fokussierungslinsen oder gekrümmte Spiegel oder durch elektronische Fokussierung oder einer Mischform aus mechanischer und elektronischer Fokussierung erzeugt werden. Ebensogut können zur unterschiedlichen Fokussierung die einzelnen Sektorabtaster 2, 3, 4 auch mit unterschiedlichen Betriebsfrequenzen arbeiten. Die Apertur der Sektorabtaster 2, 3, 4 ist an die Tiefenlage der Fokusse angepaßt, um konstanten Fokussierungsgrad bzw. gleichmäßige Tiefenschärfe zu gewährleisten.

Zur Erzeugung der Sektorabtastfelder können die Sektorabtaster 2, 3, 4 eine Schwenkbewegung ausführen, wie dies z. B. in der Figur 1 für die einzelnen Sektorabtaster 2, 3 und 4 durch die Schwenkpfeile 9, 10 und 11 sowie zugehörigen (strichpunktiert dargestellten) Begrenzungslinien 12 bis 17 des jeweils zugeordneten Sektorfeldes dargestellt ist. Die einzelnen Sektorabtaster 2, 3 und 4 können jedoch selbstverständlich auch rundum rotierende Abtaster sein, wie dies z. B. in der Figur 1 für den Sektorabtaster 3 durch die punktierte Pfeillinie 18 (bzw. auch in den Figuren 2 und 3) angedeutet ist. Bei beiden Ausführungsbeispielen rotieren die Sektorabtaster 2, 3, 4 zusammen mit der Ringscheibe 1 langsam um das Untersuchungsobjekt 8. Die Scheibenrotationsfrequenz ist sehr viel niedriger als die Frequenz, mit der die Sektorabtaster 2, 3, 4 geschwenkt oder gedreht werden. So beträgt z. B. die Drehgeschwindigkeit der Ringscheibe 1 zusammen mit den Sektorabtastern 2, 3, 4 etwa 0,1 Hz. Die Schwenk- oder Drehfrequenz der Sektorabtaster 2, 3, 4 liegt hingegen bei 3 bis 4 Hz.

Die Anordnung der Figur 1 ermöglicht Ultraschall-Reflexions-Tomographie bei ausgezeichneter Bildauflösung. Wird die Anordnung, wie in der Figur 1 angedeutet, auch noch durch ein Empfangs-Array (z. B. gekrümmtes Empfangs-Array 19 oder geradliniges Empfangs-Array 20) mit einer Vielzahl von Empfangswandlerelementen 21 (PVF$_2$) ergänzt, das dem Sektorabtaster 3 gegenüberliegt und das zusammen mit diesem um das Untersuchungsobjekt 8 rotiert, so ergibt sich die Möglichkeit der Umschaltung zwischen Reflexions-Tomographie und Transmissions-Tomographie. Im Unterschied zum Transmissions-Tomographiegerät nach der US-PS 41 05 018 ist allerdings der Ultraschall-Sender 3 im vorliegenden Falle ein mechanischer Sektor-Schallkopf, der vom Sende -Echo-Betrieb auf reinen Transmissionsbetrieb umgeschaltet ist. Außerdem sollte der Sektorabtaster 3 im Transmissionsbetrieb auch noch linienfokussiert sein.

Die Figur 2 zeigt eine erfindungsgemäße Ausführungsform in perspektivischer Darstellung. Der Rotationsring 1 besteht dabei aus insgesamt zwei Ringteilen 30 und 31, die mittels Hydraulikelementen 32, 33 in der Höhe relativ zueinander verstellbar sind. Die Hydraulikelemente 32, 33 sind mittels eines Motors 34 antreibbar. Auf dem oberen Ringteil 31 sitzen in dosenförmigen Gehäusen 35, 36 und 37 die Ultraschall-Sektorabtaster 2, 3 und 4. Die Dosengehäuse sind in Richtung der Pfeile 38, 39 und 40 um Rotationsachsen 41, 42, 43 zur Erzeugung der Sektor-Abtastfelder drehbar. Die gesamte Anordnung sitzt in einem wassergefüllten Gehäuse 44. Dieses beinhaltet ein Formgebungselement 44', z. B. aus Folienmaterial, in dem das zu untersuchende Objekt 8, d. h. im vorliegenden Falle die weibliche Brust, mit besonderer Formvorgabe (z. B. gestreckte oder gepreßte oder auch schlaffe Form) untergebracht wird. Das Gehäuse 44 ist um eine Rotationsachse 45 drehbar. Dazu dient ein Drehantriebsmotor 46 mit Antriebswelle 47 und Antriebszahnrad 48. Das Antriebszahnrad 48 greift in einen Zahnkranz 49 am Umfang des unteren Rotationsscheibenteiles 30. Dadurch wird das Gehäuse 44 mit der Gesamtanordnung der Ultraschall-Sektorabtaster 2, 3, 4 in Rotation versetzt.

Die Anordnung der Figuren 1 und 2 umfaßt unterschiedlich fokussierte mechanische Sektorabtaster 2, 3, 4 die am Umfang eines Rotationsringes 1 bzw. 30, 31 gegeneinander versetzt angeordnet sind. Selbstverständlich können einige oder auch alle Sektorabtaster 2, 3, 4 mit unterschiedlicher Frequenz (einschließlich eines separaten linienfokussierten Sektorabtasters als Sender im Transmissionsbetrieb) dicht benachbart in einem einzigen gemeinsamen Gehäuse untergebracht sein, das zusammen mit dem Rotationsring rotiert. Diese Ausführungsmöglichkeit ist in der Figur 3 dargestellt, wo die Sektorabtaster 2, 3 und 4 zusammen mit einem separaten Transmissionssender 50 in einer gemeinsamen Gehäusedose 51 angeordnet sind, die auf einem sich drehenden Rotationsring 52 zur Sektorabtastung rotiert.

Mit den bisher beschriebenen Anordnungen erhält man speziell Koronarschnitte, d. h. im Falle der Brustabtastung Schnitte parallel zur Brustwand. Sagittalschnitte, d. h. Schnitte senkrecht zur Brustwand, kann man zusätzlich erhalten, wenn weitere Ultraschall-Sektorabtaster mit ihren Sektor-Abtastebenen senkrecht zu den vorhergehenden am Rotationskörper angeordnet werden. In der Figur 2 ist eine solche Möglichkeit mit einem zusätzlichen Sektorabtaster 53 in einer Gehäusedose 54, die in Richtung des Pfeiles 55 um die Rotationsachse 56 drehbar ist, angedeutet.

Die beschriebenen Ausführungsbeispiele haben lediglich exemplarischen Charakter. Sie lassen sich im Rahmen der Erfindung beliebig modifizieren. So kann beispielsweise die Zahl der Sektorabtaster insgesamt variiert werden. Auch ihre Anordnung an der Drehvorrichtung kann so sein, daß jede sich drehende Dose z. B. in einem eigenen ultraschalldurchlässigen und wassergefüllten Gehäuse (z. B. aus Gummi) sitzt, das drehstarr an der Drehvorrichtung befestigt ist, wie dies z. B. in Figur 3 mit 57 angedeutet ist. Die dünne Wasserschicht 58 zwischen Gehäuse 57 und rotierender Dose 51 hält die Gesamtverwirbelung von Wasser gering.

## Patentansprüche

1. Ultraschall-Tomographiegerät mit einem Ultraschall-Sende/Empfangssystem, das zur Abtastung um ein Untersuchungsobjekt drehbar angeordnet ist, dadurch gekennzeichnet, daß das Ultraschall-Sende/Empfangssystem eine Mehrzahl von nach dem Sende-/Echoverfahren arbeitenden Sektorabtastern (2, 3, 4 ; 53) umfaßt, die auf unterschiedliche Objekttiefen fokussierbar (F1, F2, F3) sind.

2. Ultraschall-Tomographiegerät nach Anspruch 1, dadurch gekennzeichnet, daß neben den nach dem Sende-/Echoverfahren arbeitenden Sektorabtastern (2, 3, 4 ; 53) ein Ultraschall-Transmissionssender (3 ; 50) vorhanden ist, dem zum Transmissions-Tomographie-Betrieb ein Empfänger (19, 20), vorzugsweise Empfangs-Array, diametral gegenüberliegend zugeordnet ist.

3. Ultraschall-Tomographiegerät nach Anspruch 2, dadurch gekennzeichnet, daß der Transmissionssender (3 ; 50) ebenfalls ein Sektorabtaster ist.

4. Ultraschall-Tomographiegerät nach Anspruch 2 und 3, dadurch gekennzeichnet, daß der Sektorabtaster (50) für Transmissions-Tomographie zusätzlich zu den nach dem Sende/Echoverfahren arbeitenden Sektorabtastern (2, 3, 4) vorhanden ist.

5. Ultraschall-Tomographiegerät nach Anspruch 2 und 3, dadurch gekennzeichnet, daß der Sektorabtaster für Transmissions-Tomographie einer der Sektorabtaster (3) für die Reflexions-Tomographie ist, der vom Sende/Echoverfahren auf Transmissionsverfahren umschaltbar ist.

6. Ultraschall-Tomographiegerät nach einem der Ansprüche 3 bis 5, dadurch gekennzeichnet, daß der als Transmissionssender (3 oder 50) arbeitende Sektorabtaster linienfokussiert ist.

7. Ultraschall-Tomographiegerät nach einem der Ansprüche 1 bis 6, dadurch gekennzeichnet, daß die nach dem Sende-/Echoverfahren arbeitenden und unterschiedlich fokussierten Sektorabtaster (2, 3, 4 bzw. auch 53) sowie gegebenenfalls ein Ultraschall-Transmissionssender (50) am Umfang eines Rotationselementes (1) gegeneinander versetzt angeordnet sind.

8. Ultraschall-Tomographiegerät nach einem der Ansprüche 1 bis 6, dadurch gekennzeichnet, daß einige oder auch alle nach dem Sende-/Echoverfahren arbeitenden und unterschiedlich fokussierten Sektorabtaster (2, 3, 4) gegebenenfalls zusammen mit einem Ultraschall-Transmissionssender (50) dicht benachbart in einem gemeinsamen Gehäuse (51), das auf einem Rotationskörper (52) drehbar montiert ist, angeordnet sind.

9. Ultraschall-Tomographiegerät nach einem der Ansprüche 1 bis 8, dadurch gekennzeichnet, daß die Sektorabtaster (2, 3, 4 ; 50, 53) zur Erzeugung von Koronarschnitten und/oder Sagittalschnitten des Untersuchungsobjektes (8) ausgebildet und angeordnet sind.

10. Ultraschall-Tomographiegerät nach Anspruch 1, dadurch gekennzeichnet, daß die Sektorabtaster (2, 3, 4 ; 53) gegeneinander versetzt auf einer Drehvorrichtung (1 ; 30, 31 ; 52) angeordnet und mit dieser um das Untersuchungsobjekt (8) drehbar sind, wobei die Drehgeschwindigkeit kleiner als die Sektorabtastgeschwindigkeit ist (Fig. 1 und 2).

11. Ultraschall-Tomographiegerät nach Anspruch 1, dadurch gekennzeichnet, daß die Sektorabtaster (2, 3, 4) dicht benachbart in einem gemeinsamen Gehäuse (51) angeordnet sind, daß das Gehäuse (51) auf einem Rotationskörper (52) drehbar montiert ist, und daß das Gehäuse (51) zusammen mit dem Rotationskörper (52) um das Untersuchungsobjekt (8) drehbar ist, wobei die Drehgeschwindigkeit kleiner als die Sektorabtastgeschwindigkeit ist (Fig. 3).

**Claims**

1. An ultrasonic tomography device with an ultrasonic transmitting/receiving system which, for purposes of scanning, is arranged to be rotatable about an object to be investigated, characterised in that the rotatable ultrasonic transmitting/receiving system comprises a plurality of sector scanners (2, 3, 4 ; 53) which operate in accordance with the echo transmission method and which focus to different object depths (F1, F2, F3).

2. An ultrasonic tomography device as claimed in Claim 1, characterised in that in addition to the sector scanners (2, 3, 4 ; 53) which operate in accordance with the echo transmission method, an ultrasonic transmission transmitter (3 ; 50) is provided which, is assigned a diametrically opposed receiver (19, 20), preferably a receiving array, for transmission tomography operation.

3. An ultrasonic tomography device as claimed in Claim 2, characterised in that the transmission transmitter (3 ; 50) is likewise a sector scanner.

4. An ultrasonic tomography device as claimed in Claim 2 and 3, characterised in that the sector scanner (50) for transmission tomography is provided in addition to the sector scanners (2, 3, 4) which operate in accordance with the echo transmission method.

5. An ultrasonic tomography device as claimed in Claim 2 and 3, characterised in that the sector scanner for transmission tomography is one of the sector scanners (3) for the reflection tomography, which can be switched over from the echo transmission method to the transmission method.

6. An ultrasonic tomography device as claimed in one of Claims 3 to 5, characterised in that the sector scanner which operates as transmission transmitter (3 or 50) is line focussed.

7. An ultrasonic tomography device as claimed in one of Claims 1 to 6, characterised in that the sector scanners (2, 3, 4 and also 53) which operate in accordance with the echo transmission method are differently focussed, and possibly also an ultrasonic transmission transmitter (50), are arranged offset from one another on the periphery of a rotation element (1).

8. An ultrasonic tomography device as claimed in one of Claims 1 to 6, characterised in that some or all of the sector scanners (2, 3, 4) which operate in accordance with the echo transmission method and are differently focussed, possibly together with an ultrasonic transmission transmitter (50), are arranged closely beside one another in a common housing (51) mounted on a rotational body (52) so as to be rotatable.

9. An ultrasonic tomography device as claimed in one of Claims 1 to 8, characterised in that the sector scanners (2, 3, 4 ; 50, 53) are designed and arranged so as to produce coronary sections and/or sagittal sections of the object (8) which is to be investigated.

10. An ultrasonic tomography device as claimed in Claim 1, characterised in that the sector scanners (2, 3, 4 ; 53) are arranged offset from one another on a rotating device (1 ; 30, 31 ; 52) to be rotated by means of the rotating device about the object (8) to be investigated, the rotation speed being lower than the sector scanning speed (Fig. 1 and 2).

11. An ultrasonic tomography device as claimed in Claim 1, characterised in that the sector scanners (2, 3, 4) are arranged closely adjacent one another in a common housing (51) that is mounted on a rotational body (52) so as to be rotatable, and that the housing (51), together with the rotational body (52), can rotate about the object (8) to be investigated at a rotating speed lower than the sector scanning speed (Fig. 3).

**Revendications**

1. Appareil de tomographie par ultrasons avec un système d'émission/réception à ultrasons disposé, pour le balayage, de manière à pouvoir tourner autour de l'objet à examiner, caractérisé par le fait que le système d'émission/réception à ultrasons comporte plusieurs dispositifs de balayage sectoriel (2, 3, 4 ; 53) opérant selon le procédé émission/écho et susceptibles d'être focalisés (F1, F2, F3) sur des profondeurs diffé- rentes de l'objet.

2. Appareil de tomographie par ultrasons selon la revendication 1, caractérisé par le fait qu'en plus du dispositif de balayage sectoriel (2, 3, 4 ; 53) opérant selon le procédé émission/écho, il est prévu un émetteur à transmission ultrasonore (3 ; 50) auquel est associé, pour le fonctionnement en tomographie à transmission, diamétralement en face, un récepteur (19, 20), de préférence un système de réception.

3. Appareil de tomographie par ultrasons selon la revendication 2, caractérisé par le fait que l'émetteur à transmission (3 ; 50) est également un dispositif de balayage sectoriel.

4. Appareil de tomographie par ultrasons selon la revendication 2 et 3, caractérisé par le fait que le dispositif de balayage sectoriel (50) est prévu, pour la tomographie à transmission, en plus des dispositifs de balayage sectoriel (2, 3, 4) opérant selon le procédé émission/écho.

5. Appareil de tomographie par ultrasons selon la revendication 2 et 3, caractérisé par le fait que le dispositif de balayage sectoriel pour la tomographie à transmission est l'un des dispositifs de balayage sectoriel (3) pour la tomographie à réflexion, susceptible d'être commuté du procédé émission/écho sur le procédé à transmission.

6. Appareil de tomographie par ultrasons selon l'une des revendications 3 à 5, caractérisé par le fait que le dispositif de balayage sectoriel opérant comme émetteur à transmission (3 ou 50) est focalisé linéairement.

7. Appareil de tomographie selon l'une des revendications 1 à 6, caractérisé par le fait que les dispositifs de balayage sectoriel (2, 3, 4 et aussi 53) opérant selon le procédé émission/écho et focalisés de façons différentes, ainsi qu'éventuellement un émetteur à transmission ultrasonore (50), sont disposés avec décalage mutuel sur la périphérie d'un élément à rotation (1).

8. Appareil de tomographie par ultrasons selon l'une des revendications 1 à 6, caractérisé par le fait que certains ou même tous les dispositifs de balayage sectoriel (2, 3, 4) qui opèrent selon le procédé émission/écho et qui sont focalisés de façons différentes, sont disposés, éventuellement avec un émetteur à transmission ultrasonore (50) très près les uns des autres dans un boîtier commun (52) qui est monté à rotation sur un corps de rotation (1).

9. Appareil de tomographie par ultrasons selon l'une des revendications 1 à 8, caractérisé par le fait que les dispositifs de balayage sectoriel (2, 3, 4, 50 ; 53) sont réalisés et disposés de façon à produire des coupes coronaires et/ou des coupes sagittales de l'objet à examiner (8).

10. Appareil de tomographie par ultrasons selon la revendication 1, caractérisé par le fait que les dispositifs de balayage sectoriel (2, 3, 4 ; 53) sont disposés, avec décalage mutuel, sur un dispositif de rotation (1 ; 30, 31 ; 52) et sont susceptibles d'être mis en rotation, avec ledit dispositif de rotation, autour de l'objet à examiner (8), la vitesse de rotation étant inférieure à la vitesse de balayage sectoriel (figures 1 et 2).

11. Appareil de tomographie par ultrasons selon la revendication 1, caractérisé par le fait que les dispositifs de balayage sectoriel (2, 3, 4) sont disposés, très près l'un de l'autre, dans un boîtier commun (51), que le boîtier (51) est monté à rotation sur un corps de rotation (52), et que le boîtier (51) est susceptible, avec le corps de rotation (52), d'être entraîné en rotation autour de l'objet à examiner (8), la vitesse de rotation étant inférieure à la vitesse de balayage sectoriel (figure 3).

FIG 1

FIG 2

FIG 3